# EUROPÄISCHE PATENTSCHRIFT

(11) **EP 1 473 507 B1**
(45) Veröffentlichungstag und Bekanntmachung des Hinweises auf die Patenterteilung: **23.05.2012**
(21) Anmeldenummer: 03009639.0
(22) Anmeldetag: 29.04.2003
(51) Int. Cl.: F16M 11/04, A61B 19/00

(54) **Handgriff eines Stativkopfs und Stativkopf mit einem solchen Handgriff**
Support head handle and support head with such a handle
Poignée de tête de support et tête de support ayant une telle poignée

(43) Veröffentlichungstag der Anmeldung: 03.11.2004
(73) Patentinhaber: TRUMPF Medizin Systeme GmbH + Co. KG, 07318 Saalfeld (DE)
(72) Erfinder: Körner, Arno, 82211 Herrsching (DE)
(74) Vertreter: Hofer, Dorothea

(56) Entgegenhaltungen:
- EP-A- 1 016 820
- DE-A- 3 243 709
- DE-C- 4 200 654
- DE-U- 9 308 400
- FR-A- 2 820 971
- US-A- 4 188 682

## Beschreibung

Die vorliegende Erfindung bezieht sich auf einen Stativkopf für ein medizinisches Deckenstativ mit einem Handgriff und auf den Handgriff des Stativkopfs.

Es sind medizinische Deckenstative bekannt, die zur hängenden Aufnahme von medizinischen Geräten dienen, wie beispielsweise Überwachungsmonitoren, Beatmungssystemen, Spritzenpumpen etc.. Sie werden beispielsweise in Operationssälen oder Intensivräumen etc. für die Unterbringung der für Operationen, die Intensivpflege bzw. Untersuchung eines Patienten notwendigen Systeme verwendet.

Deckenstative bestehen aus mindestens einer vertikalen Säule und einem horizontalen schwenkbaren Arm, die über eine Drehverbindung drehbar an der Decke befestigt sind, und einem Geräteträger, auch Stativkopf genannt, in dem die notwendigen Geräte untergebracht sind. Durch den drehbar gelagerten Arm läßt sich der Stativkopf in einem gewünschten Radius verschwenken, wodurch der Zugang zum Patienten erleichtert wird. Der Stativkopf weist eine Vorderseite auf, von der aus die in den Stativkopf eingeschobenen Geräte bedient werden und die Anschlüsse für Leitungen bzw. Schläuche zu und vom Patienten zugänglich sind. An dieser Vorderseite ist ein Handgriff angeordnet, der dazu dient, den Stativkopf in eine gewünschte Position zu verschwenken.

Der Stativkopf kann an einem höhenverstellbaren Arm angebracht sein und kann dadurch zusätzlich zur Verschwenkbarkeit in seiner Höhenposition verstellt werden, indem der Arm motorisch verstellt wird.

Der Hubantrieb wird über ein Bedienelement bzw. einen Schalter betätigt. Das Bedienelement ist in dem Handgriff vorgesehen.

Damit der Stativkopf nach dem Verschwenken in seiner gewünschten Endposition bleibt, kann eine Feststellbremse vorgesehen sein, die in ihrer Ruhestellung den Stativkopf verriegelt. Wird die Feststellbremse gelöst, läßt sich der Stativkopf per Hand verschwenken. Auch hierfür ist in dem Handgriff ein Bedienelement vorgesehen. Die Feststellbremse wird pneumatisch oder elektromagnetisch durch Schalten des Bedienelements gelöst. Wird das Bedienelement betätigt, wird die Feststellbremse gelöst und der Stativkopf kann verschwenkt werden.

Da der Handgriff an der Vorderseite des Stativkopfs angebracht ist, ist er jedoch nicht von allen Seiten zugänglich. Daher ist es je nach dem, wo sich das Bedienungspersonal gerade befindet, bisweilen umständlich, den Stativkopf in die gewünschte Position zu bringen, da sich das Bedienungspersonal zuerst zur Vorderseite des Stativkopfes bewegen muß oder diesen ergonomisch ungünstig umgreifen muß, um den Handgriff und die darin untergebrachten Bedienungselemente ergreifen und betätigen zu können.

Das Gebrauchsmuster DE-G-93 08 400.5 offenbart einen Stativkopf für ein Stativ mit Tragelementen. Die Tragelemente bestehen aus einer inneren Platte und darum, zu mindest teilweise umlaufenden rechteckigen Leisten. Die Tragelemente sind unterhalb eines Abschnitts mit darin angeordneten Steckdosen an einer zusätzlichen Säule angebracht.

Die Patentanmeldung FR-A-2 820 971 offenbart ein Medizinisches Deckenstativ mit einem bogenförmigen Handgriff. Der Griff ist mit Bedienungselementen in Form von verformbaren Membranen ausgestattet, deren Betätigung in ein elektrisches, pneumatisches oder hydraulisches Signal umgewandelt wird. Der Handgriff ist an seinen Enden an der Vorderseite einer Plattform angebracht.

Die EP-A- 1016820 offenbart eine medizinische Leuchte mit einem Stativkopf gemäß dem Oberbegriff des Anspruchs 1.

Es ist Aufgabe der vorliegenden Erfindung, einen Stativkopf leichter handhabbar zu gestalten.

Diese Aufgabe wird mit einem Stativkopf gemäß Anspruch 1 bzw. mit einem Handgriff gemäß Anspruch 17 gelöst. Vorteilhafte Weiterbildungen sind Gegenstand der Unteransprüche.

Die erfindungsgemäße Gestaltung des Handgriffs erlaubt einen ergonomisch günstigen Zugang von allen Seiten. In einer vorteilhaften Ausgestaltung der Erfindung ist der Handgriff kreisringförmig ausgebildet. Er kann aber auch eine ovale oder viereckige, jedenfalls die vertikale Achse des Stativkopfs im wesentlichen umlaufende Gestalt besitzen. Der Handgriff muß keinen geschlossenen Umlauf bilden, sondern kann auch ein- oder mehrmals unterbrochen sein, beispielsweise in der Form zweier Halbkreise mit einem dazwischenliegendem Spalt.

Durch die Weiterbildung des Handgriffs mit einem ersten von seiner inneren Umfangsoberfläche zur Mittelachse hin verlaufenden Steg, der koaxial zur Mittelachse eine Nabe aufweist, in der eine Befestigungsstange befestigt ist, kann der Handgriff an einer Ober- oder Unterseite des Stativkopfs befestigt werden.

Wenn in vorteilhafterweise ein zweiter von seiner inneren Umfangsoberfläche zur Mittelachse hin verlaufender Steg vorgesehen ist, kann die Stabilität des Handgriffs noch erhöht werden, insbesondere wenn die beiden Stege diametral gegenüberliegend angeordnet sind.

Gemäß einer vorteilhaften Weiterbildung kann der Handgriff nicht durchgehend umlaufend ausgebildet sein, sondern aus zwei Teilen bestehen, die jeweils in etwa einen Zugangsbereich von 180° abdecken. Jeder Teil des Handgriffs ist an einem der Stege befestigt.

Gemäß einer vorteilhaften Weiterbildung ist der Handgriff mit mindestens zwei Bedienelementeaufnahmen versehen, die zur Aufnahme von Bedienelementen dienen, mit denen beispielsweise eine Feststellbremse des Stativkopfs gelöst werden kann, oder ein Motor betätigt werden kann, der den Stativkopf nach oben oder nach unten versetzt. Dadurch kann der Stativkopf in jede gewünschte Position gebracht werden.

Das Vorsehen von wenigstens drei, vorzugsweise vier in Umfangsrichtung gleichmäßig beabstandeten Bedienelementeaufnahmen mit darin angeordneten Bedienelementen schafft die Möglichkeit, die Bedienelemente von allen Seiten des Stativkopfes aus ergonomisch günstig betätigen zu können.

Bei Bedarf ist jeder Bedienelementeaufnahme noch eine zweite Bedienelementeaufnahme zugeordnet, falls die Zahl der benötigten Bedienelemente höher ist.

Gemäß einer vorteilhaften Ausgestaltung der Erfindung ist die Nabe, die Befestigungsstange und der Handgriff selbst hohl ausgebildet, so daß Kabel durchgeführt werden können, die an die Bedienelemente angeschlossen werden.

Nachfolgend wird die Erfindung anhand einer derzeit bevorzugten Ausführungsform unter Bezugnahme auf die beigefügten Figuren näher erläutert.

Fig. 1 ist eine perspektivische Ansicht eines erfindungsgemäßen Handgriffs.

Fig. 2 ist eine Draufsicht des Handgriffs aus Fig. 1.

Fig. 3 ist eine perspektivische Ansicht eines Stativkopfs mit dem Handgriff aus Fig. 1.

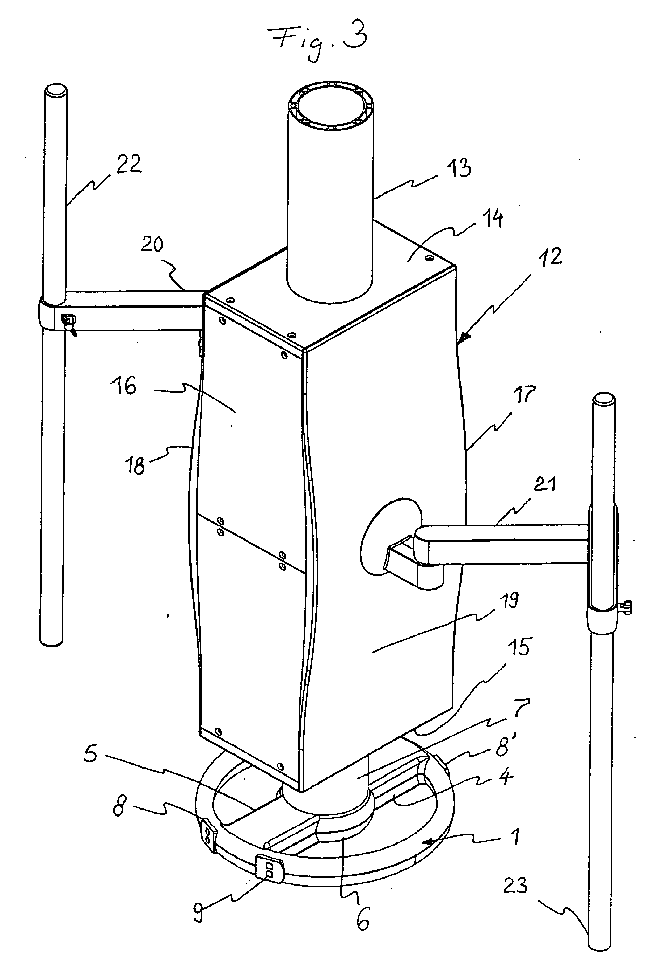

In Fig. 3 ist ein Stativkopf 12 der vorliegenden Ausführungsform gezeigt. Der Stativkopf 12 weist eine Oberseite 14, eine Unterseite 15, eine Vorderseite 16, eine Rückseite 17, eine linke Seite 18 sowie eine rechte Seite 19 auf. Der Stativkopf 12 ist in der Mitte seiner Oberseite 14 an einer vertikal verlaufenden Säule 13 befestigt. Die Säule ist mit einem hier nicht dargestellten schwenkbaren Arm verbunden. An der linken und der rechten Seite 18, 19 des Stativkopfs sind jeweils Stangenhaltearme 20, 21 angebracht, die Stangen 22, 23 halten. An diesen Stangen können verschiedene Geräte befestigt werden, beispielsweise mehrere Spritzenpumpen. An der Vorder- und Rückseite des Stativkopfs 12 sind Anschlußmöglichkeiten wie Stromsteckdosen, Gassteckdosen etc. vorgesehen, die hier jedoch nicht dargestellt sind. Diese dienen zum Anschluß der nicht dargestellten Spritzenpumpen etc., die an den Stangen 22, 23 befestigt sind. An der Unterseite 15 des Stativkopfs 12 ist eine Verbindungssäule 7 befestigt. Diese mündet in eine Nabe 6 eines Handgriffs 1. An der Nabe 6 sind zwei Stege 4, 5 befestigt, die sich diametral nach außen erstrecken. An den der Nabe 6 gegenüberliegenden Seiten der beiden Stege 4, 5 ist ein ringförmiger Handgriff 1 angeordnet.

Der Handgriff 1 ist aus einer oberen und einer unteren im Spritzgußverfahren hergestellten Formhälfte 2, 3 aus Kunststoff zusammengesetzt. Die beiden Formhälften sind miteinander verklebt. Der Handgriff 1 besitzt eine äußere Umfangsoberfläche 10 und eine innere Umfangsoberfläche 11. Die beiden Stege 4 und 5 sind in der vorliegenden Ausführungsform an dem ringförmigen Handgriff 1 angeformt und sind somit Teil der oberen bzw. unteren Formhälfte 2, 3. Die Nabe 6 ist ebenfalls kreisringförmig konzentrisch zum Handgriff 1 ausgebildet.

In dem ringförmigen Handgriff 1 sind Bedienelementeaufnahmen 8 ausgespart. Die Bedienelementeaufnahmen 8 sind mit angeformten vorstehenden Wandabschnitten ausgebildet, die als Fassungen zur Aufnahme von Bedienelementen 9 dienen. In der vorliegenden Ausführungsform dienen die Bedienelemente zum Lösen einer Feststellbremse und zum Ansteuern eines motorbetriebenen, in der Höhe verstellbaren, Arms. Sie können jedoch auch zur Bedienung anderer oder zusätzlicher Funktionen dienen.

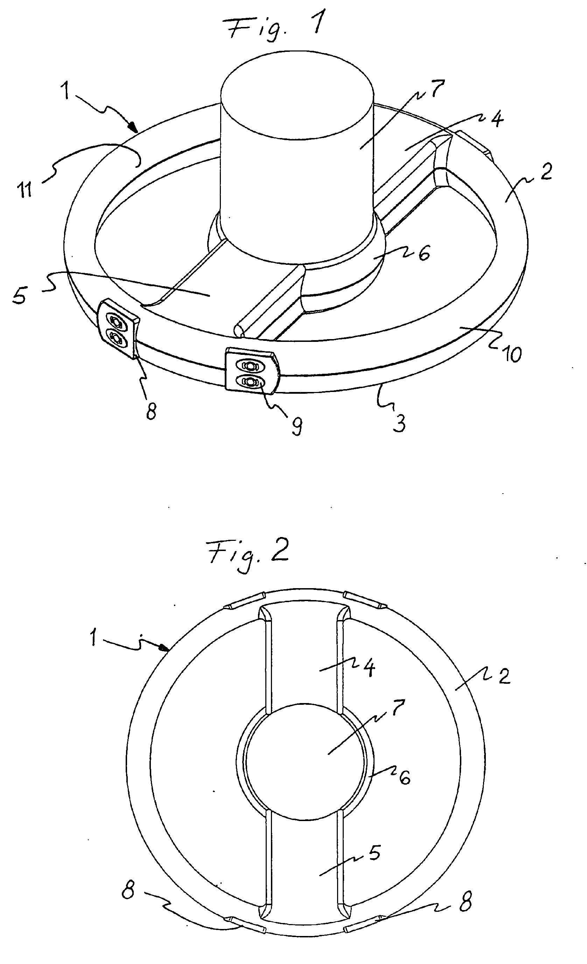

In der vorliegenden Ausführungsform sind insgesamt vier Bedienelementeaufnahmen 8 vorgesehen, zwei an der Vorderseite 16 des Stativkopfs 12, zwei an der Rückseite 17. Wie in Fig. 1 gezeigt, sind je zwei Bedienelementeaufnahmen 8 im Bereich der Stege 4 und 5 angeordnet. Genauer gesagt sind je zwei Bedienelementeaufnahmen so am ringförmigen Handgriff 1 angeordnet, daß das Bedienungspersonal beim Umgreifen des Handgriffs mit beiden Händen die Bedienelemente 9 ergonomisch günstig mit dem Daumen betätigen kann.

Sowohl die Verbindungssäule 7, als auch die Stege 4, 5 und der ringförmige Handgriff 1 sind hohl ausgebildet. Durch diesen Hohlraum werden die Kabel für den Anschluß der Bedienelemente geführt. Die Bedienelemente 9 bestehen in der vorliegenden Ausführungsform aus Schalter mit einer Folientastatur, die von außen auf die Bedienelementeaufnahme 8 aufgeklebt sind.

## Patentansprüche

1. Stativkopf (12) für ein medizinisches Deckenstativ, mit einer vertikalen Mittelachse und einem im wesentlichen die vertikale Achse umlaufend ausgebildeten Handgriff, der eine äußere und eine innere Umfangsoberfläche (10, 11) aufweist, **dadurch gekennzeichnet, daß** eine Nabe (6) vorgesehen ist, von der sich ein erster Steg (4, 5) nach außen zum Handgriff erstreckt.

2. Stativkopf (12) nach Anspruch 1, **dadurch gekennzeichnet, daß** sich der Steg (4, 5) von der inneren Umfangsoberfläche (11) des Handgriffs (1) zur Nabe (6) erstreckt.

3. Stativkopf (12) nach Anspruch 2, **dadurch gekennzeichnet, daß** der Handgriff (1) einen zweiten von seiner inneren Umfangsoberfläche (11) zur Nabe (6) verlaufenden Steg (4, 5) aufweist.

4. Stativkopf (12) nach einem der Ansprüche 1 bis 3, **dadurch gekennzeichnet, daß** die Nabe (6) hohl ausgebildet ist.

5. Stativkopf (12) nach Anspruch 3, **dadurch gekennzeichnet, daß** der umlaufende Handgriff (1) aus zwei getrennten Teilen besteht, von denen jeder an je einem Steg (4, 5) befestigt ist.

6. Stativkopf (12) nach Anspruch 4 oder 5, **dadurch gekennzeichnet, daß** in der Nabe (6) eine Verbindungssäule (7) befestigt ist.

7. Stativkopf (12) nach Anspruch 6, **dadurch gekennzeichnet, daß** auch die Verbindungssäule (7) zur Durchführung von Kabeln hohl ausgebildet ist.

8. Stativkopf (12) nach einem der vorhergehenden Ansprüche, **dadurch gekennzeichnet, daß** zwei Bedienelementeaufnahmen (8) in dem Handgriff (1) im Wesentlichen diametral gegenüberliegend angeordnet sind.

9. Stativkopf (12) nach einem der vorhergehenden Ansprüche 1 bis 7, **dadurch gekennzeichnet, daß** vier Bedienelementeaufnahmen (8) mit einem Winkelabstand von im Wesentlichen 90° in dem Handgriff (1) angeordnet sind.

10. Stativkopf (12) nach einem der vorhergehenden Ansprüche 8 oder 9, **dadurch gekennzeichnet, daß** jeder Bedienelementeaufnahme (8) eine zweite Bedienelementeaufnahme in einem solchen Abstand zugeordnet ist, daß bei Umgreifen des Handgriffs (1) mit zwei Händen, der Daumen einer jeden Hand eine Bedienelementeaufnahme (8) erreicht.

11. Stativkopf (12) nach einem der vorhergehenden Ansprüche 8 bis 10, **dadurch gekennzeichnet, daß** in jeder Bedienelementeaufnahme (8) ein oder mehrere Bedienelement(e) (9) angeordnet ist (sind).

12. Stativkopf (12) nach dem vorhergehenden Anspruch, **dadurch gekennzeichnet, daß** jedes Bedienelement (9) einen Schalter zum Lösen einer Feststellbremse und/oder einen Schalter zum Betätigen eines höhenverstellbaren Arms aufweist.

13. Stativkopf (12) nach einem der vorhergehenden Ansprüche, **dadurch gekennzeichnet, daß** der Handgriff auf der Ober- oder Unterseite des Stativkopfs (12) befestigt ist.

14. Stativkopf (12) nach einem der vorhergehenden Ansprüche, **dadurch gekennzeichnet, daß** der Handgriff (1) so ausgebildet ist, daß er den Stativkopf (12) umgibt, so daß der Stativkopf die Nabe und die Befestigungsstange bildet.

15. Stativkopf (12) nach einem der vorhergehenden Ansprüche, **dadurch gekennzeichnet, daß** der Handgriff (1) ringförmig ausgebildet ist.

16. Stativkopf (12) nach einem der vorhergehenden Ansprüche, **dadurch gekennzeichnet, daß** der Handgriff (1) hohl ausgebildet ist.

17. Handgriff (1) eines Stativkopfs (12) eines medizinischen Deckenstativs mit einer vertikalen Mittelachse, wobei der Handgriff (1) die vertikale Mittelachse des Deckenstativs (12) im wesentlichen umlaufend ausgebildet ist und eine äußere und eine innere Umfangsoberfläche (10, 11) aufweist, **dadurch gekennzeichnet, daß** sich von der inneren Umfangsoberfläche (11) ein Steg (4, 5) in Richtung Mittelachse erstreckt und in eine Nabe (6) mündet, in der eine Verbindungssäule (7) befestigt ist, die sich vertikal erstreckt und mit dem Stativkopf (12) verbindbar ist, wobei die Verbindungssäule (7), der Steg (4, 5) und der Handgriff (1) Hohlräume aufweisen, und an der Außenumfangsoberfläche (10) Bedienelemente (9) ausgebildet sind.

## Claims

1. Support head (12) for a medical ceiling mounted support system, with a vertical central axis and a handle formed substantially surrounding the vertical axis, comprising an outer and an inner circumferential surface (10, 11), **characterized in that** a first hub (6) from which a first web (4, 5) extends outwardly to the handle is provided.

2. Support head (12) according to claim 1, **characterized in that** the web (4, 5) extends from the inner circumferential surface (11) of the handle (1) to the hub (6).

3. Support head (12) according to claim 2, **characterized in that** the handle (1) comprises a second web (4, 5) running from the inner circumferential surface (11) of the handle (1) to the hub (6).

4. Support head (12) according to any of claims 1 to 3, **characterized in that** the hub (6) is hollowly formed.

5. Support head (12) according to claim 3, **characterized in that** the surrounding handle (1) consists of two separate parts each being fixed at a respective web (4, 5).

6. Support head (12) according to claim 4 or 5, **characterized in that** a connection column (7) is fixed in the hub (6).

7. Support head (12) according to claim 6, **characterized in that** the connection column (7) is also hollowly formed for conducting cables.

8. Support head (12) according to any of the preceding claims, **characterized in that** two operating member seatings (8) are arranged diametrically opposite in the handle (1).

9. Support head (12) according to any of the preceding claims 1 to 7, **characterized in that** four operating member seatings (8) are arranged in the handle (1) at an angle of substantially 90°.

10. Support head (12) according to any of the preceding claims 8 or 9, **characterized in that** a second operating member seating is allocated to each operating member seating (8) in such a distance that the thumb of each hand reaches an operating member seating (8) when encompassing the handle (1) with two hands.

11. Support head (12) according to any of the preceding claims 8 to 10, **characterized in that** one or multiple operating member(s) (9) is (are) arranged in each operating member seating (8).

12. Support head (12) according to any of the preceding claims, **characterized in that** each operating member (9) comprises a switch for releasing a fixing brake and/or a switch for operating a height-adjustable arm.

13. Support head (12) according to any of the preceding claims, **characterized in that** the handle is fixed on the upper side or the lower side of the support head (12).

14. Support head (12) according to any of the preceding claims, **characterized in that** the handle (1) is formed such that it surrounds the support head (12) so that the support head forms the hub and the connection rod.

15. Support head (12) according to any of the preceding claims, **characterized in that** the handle (1) is formed in the shape of ring.

16. Support head (12) according to any of the preceding claims, **characterized in that** the handle (1) is hollowly formed.

17. Handle (1) of a support head (12) of a ceiling mounted medical support system having a vertical central axis, wherein the handle (1) is formed such that it substantially surrounds the vertical central axis of the support system (12) and the handle (1) comprises an outer and an inner circumferential surface (10, 11), **characterized in that** a web (4, 5) extends from the inner circumferential surface (11) towards the central axis and runs into a hub (6) in which a connection column (7) vertically extending and connectable to the support head (12) is fixed, wherein the connection column (7), the web (4, 5) and the handle (1) comprise cavities, and wherein operating members (9) are formed at the outer circumferential surface (10).

## Revendications

1. Tête de statif (12) pour un statif plafonnier médical, avec un axe vertical central et une poignée réalisée de manière à entourer sensiblement l'axe vertical et présentant une surface périphérique extérieure et une surface périphérique intérieure (10, 11), **caractérisée en ce qu'**un moyeu (6) est prévu, à partir duquel une première traverse (4, 5) s'étend vers l'extérieur jusqu'à la poignée.

2. Tête de statif (12) selon la revendication 1, **caractérisée en ce que** la traverse (4, 5) s'étend de la surface périphérique intérieure (11) de la poignée (1) vers le moyeu (6).

3. Tête de statif (12) selon la revendication 2, **caractérisée en ce que** la poignée (1) comporte une deuxième traverse (4, 5) s'étendant de sa surface périphérique intérieure (11) vers le moyeu (6).

4. Tête de statif (12) selon l'une des revendications 1 à 3, **caractérisée en ce que** le moyeu (6) est prévu creux.

5. Tête de statif (12) selon la revendication 3, **caractérisée en ce que** la poignée circulaire (1) se compose de deux parties séparées, dont chacune est fixée sur une traverse (4, 5) respective.

6. Tête de statif (12) selon la revendication 4 ou la revendication 5, **caractérisée en ce qu'**une colonne de connexion (7) est fixée dans le moyeu (6).

7. Tête de statif (12) selon la revendication 6, **caractérisée en ce que** le moyeu (6) ainsi que la colonne de connexion (7) sont prévus creux pour permettre le passage de câbles.

8. Tête de statif (12) selon l'une des revendications précédentes, **caractérisée en ce que** deux logements pour éléments de commande (8) sont ménagés dans la poignée (1), sensiblement diamétralement opposés l'un à l'autre.

9. Tête de statif (12) selon l'une des revendications 1 à 7, **caractérisée en ce que** quatre logements pour éléments de commande (8) sont ménagés dans la poignée (1) sensiblement avec un espacement angulaire de 90°.

10. Tête de statif (12) selon la revendication 8 ou la revendication 9, **caractérisée en ce qu'**un deuxième logement pour éléments de commande est affecté à chaque logement pour éléments de commande (8), et espacé de celui-ci de telle manière que le pouce de chaque main atteigne un logement pour éléments de commande (8) en cas de saisie à deux mains de la poignée (1).

11. Tête de statif (12) selon l'une des revendications 8 à 10, **caractérisée en ce qu'**un ou plusieurs éléments de commande (9) sont disposés dans chaque logement pour éléments de commande (8).

12. Tête de statif (12) selon la revendication précédente, **caractérisée en ce que** chaque élément de commande (9) comporte un commutateur pour le desserrage d'un frein d'immobilisation et/ou un commutateur pour la manoeuvre d'un bras réglable en hauteur.

13. Tête de statif (12) selon l'une des revendications précédentes, **caractérisée en ce que** la poignée est fixée sur la face supérieure ou la face inférieure de la tête de statif (12).

14. Tête de statif (12) selon l'une des revendications précédentes, **caractérisée en ce que** la poignée (1) est réalisée de manière à entourer la tête de statif (12), de telle manière que la tête de statif forme le moyeu et la barre de fixation.

15. Tête de statif (12) selon l'une des revendications précédentes, **caractérisée en ce que** la poignée (1) est prévue en forme d'anneau.

16. Tête de statif (12) selon l'une des revendications précédentes, **caractérisée en ce que** la poignée (1) est prévue creuse.

17. Poignée (1) pour une tête de statif (12) d'un statif plafonnier médical avec un axe vertical central, ladite poignée (1) étant réalisée de manière à entourer sensiblement l'axe vertical central du statif plafonnier (12) et présentant une surface périphérique extérieure et une surface périphérique intérieure (10, 11), **caractérisée en ce qu'**une traverse (4, 5) s'étend de la surface périphérique intérieure (11) dans la direction de l'axe central et débouche dans un moyeu (6) où est fixée une barre de fixation (7) qui s'étend verticalement et peut être raccordée à la tête de statif (12), la barre de fixation (7), la traverse (4, 5) et la poignée (1) présentant des cavités, et des éléments de commande (9) étant réalisés sur la surface périphérique extérieure (10).
